# EUROPEAN PATENT SPECIFICATION

(11) **EP 0 866 684 B1**
(45) Date of publication and mention of the grant of the patent: **16.08.2001**
(21) Application number: 96938210.0
(22) Date of filing: 11.11.1996
(51) Int. Cl.: A61K 7/06

(54) **COSMETIC HAIR TREATMENT METHOD**
VERFAHREN ZUR KOSMETISCHEN HAARBEHANDLUNG
PROCEDE DE TRAITEMENT COSMETIQUE DES CHEVEUX

(30) Priority: 16.12.1995 GB 9525775
(43) Date of publication of application: 30.09.1998
(73) Proprietor: UNILEVER PLC, London EC4P 4BQ (GB); UNILEVER N.V., 3013 AL Rotterdam (NL)
(72) Inventor: GIBSON, Walter, Thomas, F-78860 Saint-Nom-la-Bretèche (FR); PARMAR, Preyesh, Wellingborough, Northamptonshire NN8 4UJ (GB); RICHES, Caroline, Bedford MK41 0DU (GB); WESTGATE, Gillian, Elizabeth, Irthlingborough, Northampton NN9 5RZ (GB)
(74) Representative: Rots, Maria Johanna Francisca
(86) International application number: EP9605008
(87) International publication number: WO9722329

(56) References cited:
- WO-A-96/17586
- US-A- 3 242 052
- US-A- 4 201 235
- US-A- 5 223 252
- US-A- 5 241 973
- US-A- 5 470 876
- STN, File Supplier, Karlsruhe, DE, File XP002024404
- PATENT ABSTRACTS OF JAPAN vol. 96, no. 001 (C & JP 08 020518 A (SHISEIDO), 23 January 1996,

## Description

This invention relates to methods of cosmetic treatment of human hair to enhance the mechanical properties of the hair fibre. In particular the invention relates to the delivery of a composition comprising cysteine to the hair to supplement the hair roots and to cause the hair fibre to become thicker, so increasing hair body and volume.

Cysteine is an amino acid which has been recognised as linked with the growth of human hair. For example, JP 58162511 describes an oral preparation for the growth and generation of hair which is prepared from L-cysteine and other amino acids. RO 99940 describes a lotion for combatting scurf and regenerating hair comprising a blend of cysteine and methionine with inorganic sulphates and herb extracts. DE 1617477 describes a hair tonic which is said to stimulate hair roots to produce new growth which comprises 0.3% cysteine mixed with an array of other substances including vitamins, hormones, other amino acids and salicylic acid. JP 56087514 mentions that cystine or cysteine have attracted attention as promoters for the growth of hair but may cause problems due to lack of oxidation stability. Dithioglycollic acid is taught as a suitable replacement.

US 5,470,876 describes a topical pharmaceutical treatment for hair loss based on superoxide dismutase (SOD) comprising a complex of an amino acid or peptide with a transition metal.

Many formulations have been proposed over the years for the promotion of the growth of human hair or the curing or prevention of baldness. However, there is no discussion in the prior art of an effect on the mechanical properties of the existing hair fibre as such.

Increasing hair diameter leads to concomitant increases in hair bending stiffness and tensile strength. For example, the former parameter can be represented as an index calculated on the basis of the 2nd moment of area of the hair's transverse section (a concept used in the engineering industry to predict the relative bending resistance of different structures), hence the relationship to diameter. Increasing hair bending stiffness and tensile strength leads to an increase in the way the hair "stands-off" from the scalp, the property known as "root lift". Related to this is the consumer's perception of improved fullness, body and volume. The diameter of the hair has been reported by Tolgysi J.Soc.Cosm.Chem. 7. 571-578 (1976) to have a particularly strong effect on hair body.

Consequently, there exists a need for substances which may be topically supplied to the human hair or scalp to increase the diameter of a thinning hair, or to maintain the fibre diameter at its maximum potential.

Fratini et al (J. Invest Dermatol 102: 178-185, 1994) have shown that the abomasal or intravenous infusion of either cysteine or methionine into sheep consuming roughage-based (i.e. nutritionally poor) diets stimulates wool growth and increased the sulphur content of the "new" wool. Cysteine infusion usually leads to an increase in wool growth rate and an increase in fibre diameter of 5-10% , typically 1-3µm. Surprisingly, we have found that the levels of cysteine in plucked human hair roots are very low or undetectable in comparison with other amino acids, and that this deficiency is apparent irrespective of the subject's state of nutrition. We have further established, from in vitro studies using isolated human hair follicles, that the administration to the hair follicle of a composition comprising cysteine has a thickening effect on the hair fibre. It is unexpected that the mechanical properties of the normal human hair fibre would be critically dependent on supplementation with cysteine, since in the studies on sheep discussed above, the animals were initially underfed in order for the effect of cysteine or methionine supplementation to become apparent. Moreover, the cysteine or methionine was supplied to the sheep by endogenous delivery rather than exogenous application. Furthermore, the wool fibre comprises only one cuticle cell fibre layer (human hair comprises 8-10) and is designed to be shed and regenerated every year.

Accordingly, the present invention provides a cosmetic method for imparting body and volume to human hair comprising increasing the diameter of the hair fibre by supplementation of the hair root with a composition comprising between 0.01 to 10% by weight cysteine, the cysteine being delivered to the hair root by topical application of the composition to the hair and/or the scalp. The source of cysteine is a protein hydrolysate comprising amino acids in free and peptide form, wherein more than 50% by weight of the hydrolysate are free amino acids and at least 2% by weight of the hydrolysate of the amino acids are cysteine

Cysteine can be incorporated into a composition for topical application to the hair and/or scalp, for imparting body and volume to human hair.

The source of the cysteine is a protein hydrolysate. Protein hydrolysates are a source of amino acids in both free and peptide form. The amino acids are more than 50% free. The hydrolysate must have a significant cysteine content. At least 2% by weight of the amino acids in the hydrolysate are cysteine/cystine. The use of hydrolysates reduces the odour problems sometimes encountered with the sulphur containing amino acids and may reduce the extent of stimulation of dandruff and other foreign organisms on the scalp or in the region of the hair root. Furthermore, there appears to be benefit from use of a hydrolysate which is greater than the contribution expected, in terms of hair nutrition, from the individual amino acids.

The total amount of cysteine present in the composition for use in the method according to the invention is between 0.01 and 10% by weight, which upper limit is influenced by solubility considerations. Preferably the cysteine is used at a level of between 0.05 and 5% by weight, most preferably between 0.1 and 0.5% by weight.

The cysteine solution utilised in the formulation of compositions for use in the method of the invention is suitably of a concentration between 0.04 mMolar and 6 mMolar, preferably m.

The composition for use in the method according to the invention can be formulated as a shampoo and will then accordingly comprise one or more surfactant which are cosmetically acceptable and suitable for topical application to the hair.

Preferred surfactants, which may be used singularly or in combination, are selected from anionic, nonionic, amphoteric and zwitterionic surfactants, and mixtures thereof.

Suitable anionic surfactants are the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauroyl isethionate and sodium N-lauryl sarcosinate. The most preferred anionic surfactants are sodium lauryl sulphate, triethanolamine monolauryl phosphate, sodium lauryl ether sulphate 1 EO, 2EO and 3EO, ammonium lauryl sulphate and ammonium lauryl ether sulphate 1EO, 2EO and 3EO.

Nonionic surfactants suitable for use in compositions for use in the invention may include condensation products of aliphatic (C₈ - C₁₈) primary or secondary linear or branched chain alcohols or phenols with alkylene oxides, usually ethylene oxide and generally having from 6 to 30 ethylene oxide groups.

Other suitable nonionics include mono- or di-alkyl alkanolamides. Examples include coco mono- or diethanolamide and coco mono-isopropanolamide.

Further suitable nonionic surfactants are the alkyl polyglycosides (APG's). Typically, the APG is one which comprises an alkyl group connected (optionally via a bridging group) to a block of one or more glycosyl groups. Preferred APG's are defined by the following formula:

RO - (G) ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Amphoteric and zwitterionic surfactants suitable for use in compositions for use in the invention may include alkyl amine oxides, alkyl betaines, alkyl amidopropyl betaines, alkyl sulphobetaines (sultaines), alkyl glycinates, alkyl carboxyglycinates, alkyl amphopropionates, alkyllamphoglycinates, alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine and sodium cocamphopropionate.

The surfactant(s) may be present in compositions for use in the invention in a total amount of from about 1 to about 40% by weight, preferably from about 2 to about 30% by weight.

Compositions for use in the invention may also contain one or more hair conditioning agents, as are well known in the art. Such conditioning agents may include cationic surfactants, cationic polymers, volatile or non-volatile silicones and other materials which are known in the art as having desirable hair conditioning properties.

Such compositions may optionally include a deposition polymer to aid deposition of the conditioning agent(s) onto the hair. Suitable deposition polymers include cationic guar gum derivatives, cationic polyacrylamides and cationic guar gum derivatives, cationic polyacrylamides and cationic cellulose ether derivatives. Particularly preferred are those cationic guar gum derivatives given the CTFA designation guar hydroxypropyl trimonium chloride, available commercially for example as JAGUAR C13S.

As further optional components for inclusion in compositions for use in the invention, in addition to water, may be mentioned the following conventional adjunct materials known for use in cosmetic compositions: suspending agents, thickeners, pearlescing agents, opacifiers, salts, perfumes, buffering agents, colouring agents, emollients, moisturisers, foam stabilisers, sunscreen materials, antimicrobial agents, preservatives, natural oils and extracts, propellants.

The composition for use in the invention can be formulated as a shampoo or hair conditioner, or as a liquid or gel, or as a lotion having a viscosity of from 4,000 to 10,000 mPas, a fluid cream having a viscosity of from 10,000 to 20,000 mPas or a cream having a viscosity of from 20,000 to 100,000 mPas, or above.

The composition can be packaged in a container to suit its viscosity and intended use by the consumer. For example, a shampoo, conditioner, liquid, gel, lotion or fluid cream can be packaged in a bottle or a sachet or a propellant-driven aerosol device or a container fitted with a pump suitable for finger operation. When the composition is a cream, it can simply be stored in a non-deformable bottle or squeeze container, such as a tube or a lidded jar.

The compositions for use in the invention are primarily intended for topical application to the hair and/or scalp of the human subject to impart body and volume to the hair by increasing hair diameter.

The amount of the composition and the frequency of application to the hair and/or scalp can vary widely, depending on personal needs, but it is suggested as an example that topical application of from 0.1 to 5g daily containing from 0.01 to 10% by weight cysteine over the period of at least six months will in most cases result in an increase in hair diameter and hence an improvement in hair body and volume.

The invention will now be illustrated by the following nonlimiting Examples, in which:
- Figure 1: is a graph showing cumulative hair growth (mm) with time for different concentrations of cysteine;
- Figure 2: is a graph showing the up-take of cysteine by hair follicles as a function of cysteine concentration;
- Figure 3: is a graph showing the effect of cysteine reversal on cumulative hair growth (mm) with time;
- Figure 4: is a graph showing the effect of cysteine concentration on the diameter (µm) of hair fibres;
- Figure 5: is a graph showing the ratio of in vitro to in vivo hair diameter for different concentrations of cysteine; and
- Figure 6: is a graph showing the influence of cysteine availability on hair fibre diameter.

### EXAMPLES

### Evaluation of Efficacy of Cysteine using the In Vitro Hair Follicle Growth Test

The effect of cysteine on hair diameter was assessed using an in vitro test which uses isolated human hair follicles in a culture medium.

### Example 1

### Growth of Human Hair In Vitro is dependent upon the Concentration of Cysteine in the Medium

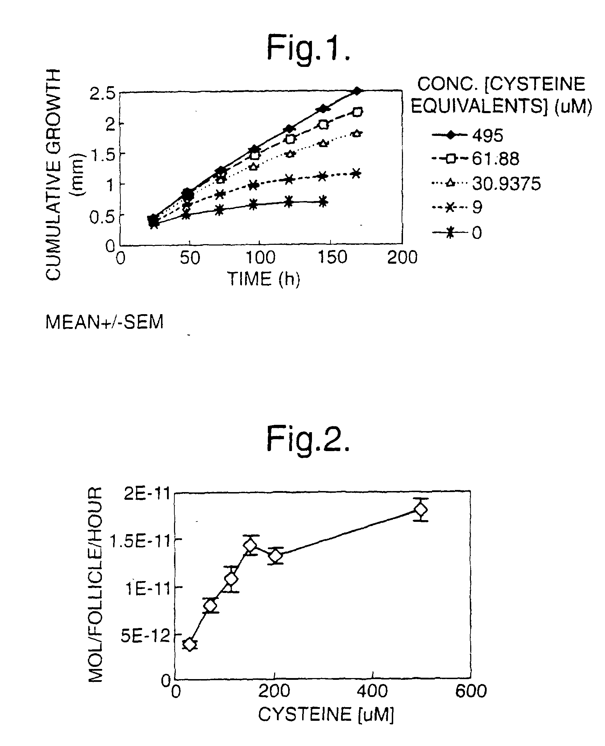

Human hair follicles were isolated from scalp skin by the methods described by Philpott et al J Cell Science 97 463-471, 1990. Follicles isolated in this way are undamaged and are capable of growth in vitro for several days at rates comparable with those described for hair growth in vivo. Follicles isolated in this way were cultured as described by Westgate et al Br. J. Dermatol 129 372-379, 1993 in a completely defined culture medium called Williams E medium (obtainable from ICN FLOW), the latter supplemented with antibiotics, glutamine, hydrocortisone and Insulin. In this example the concentration of cysteine + cystine (referred to as half cystine) was varied and the daily rate of growth of follicles measured. The results are shown in Table 1, and Figure 1 and demonstrates that growth increases with cysteine concentration in the medium.

### Example 2

### Incorporation of cvsteine into human hair in vitro is concentration dependent

Follicles were isolated as described in Example 1 and cultured for 5 hours in the presence of a range of concentrations of cysteine, a small proportion of which was radiolabelled. After 5 hours the follicles were transferred to normal supplemented Williams E medium to "chase" the radioactivity into the fibre being grown in the follicle, for 48 hours. The hair fibre was isolated from each follicle using a combination of detergent and enzyme and the 'naked' fibre dissolved and subjected to liquid scintillation counting. Results are shown in Figure 2 and Table 2.

### Example 3

### Addition of Cysteine can increase the growth rate of human hair in vitro

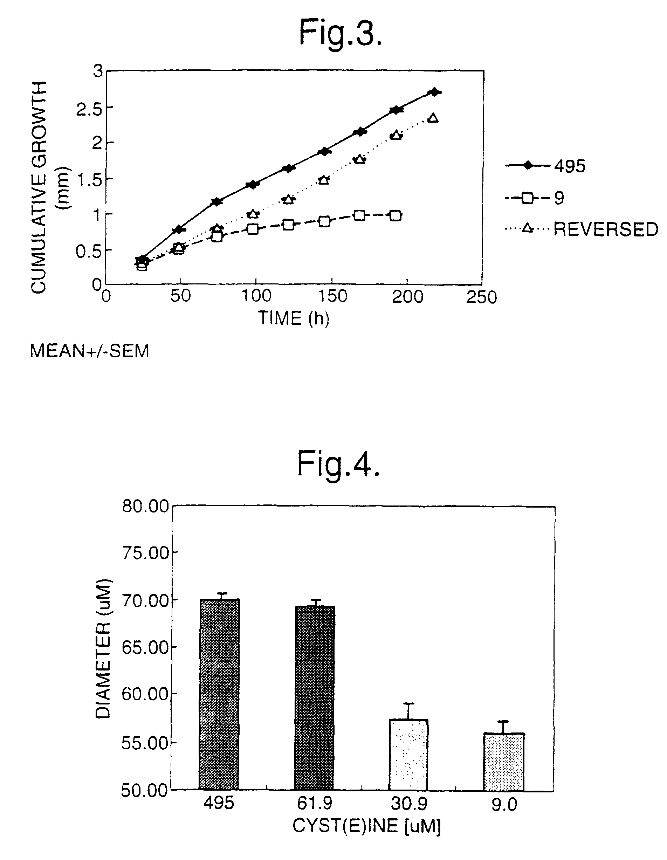

Human hair follicles were isolated as described in Example 1 and placed into culture in Williams E medium formulated to contain a lower concentration of cysteine. Follicles were cultured for 2 days in this medium then transferred to medium containing a higher amount of cysteine. Results are shown in Table 3 and Figure 3 and demonstrate that the follicles can respond to an increase in the concentration of cysteine in the medium by increasing their growth rate.

### Example 4

### The diameter of human hair fibres grown in vitro is dependent on the concentration of cysteine used in the medium

Follicles were isolated as described in Example 1 and placed into culture in supplemented Williams E medium containing a range of concentrations of cysteine. Following several days of culture(7-11), follicles were removed from the medium and the fibres isolated from the follicles as described above. The diameter of the fibres was measured using a modified Laser Scan Micrometer (Mitotoyu) coupled to a moving jig which was capable of rotating the fibre through 180° and moving the fibre through the laser beam at intervals of 0.2mm. Each isolated fibre contained a small part which was formally grown in vivo, the diameter of this "in vivo" end of the fibre. The diameter of the fibre grown in vitro was measured at intervals of 0.2mm along the fibre. For estimation of the effect of different media concentrations of cysteine on diameter, the average in vitro diameter was compared across the treatment concentrations of cysteine and the ratio of in vitro diameter to in vivo diameter was calculated.

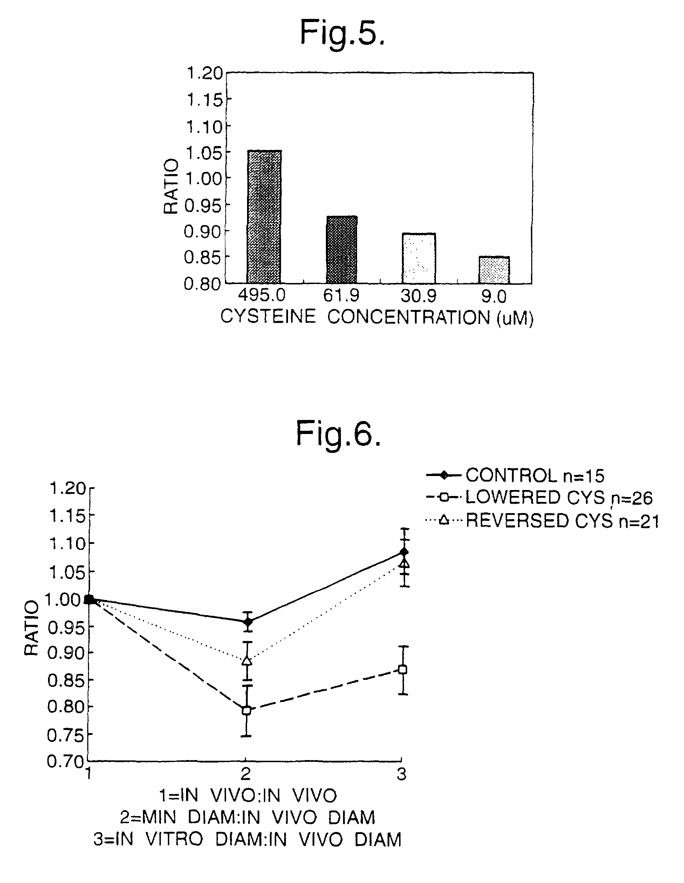

Results are shown in Table 4 and Figures 4 and 5 and demonstrate that the diameter of the fibres is dependent on the concentration of cysteine available to the follicles.

### Example 5

### Addition of cvsteine can increase the diameter of human hair in vitro

Follicles were isolated as described in Example 1 and cultured as described in Example 3. Fibres were isolated and diameter measured as described in Example 4. The results show that the diameter of the fibre has decreased from the in vivo diameter when placed in a lower concentration of cysteine (9.0µM) and increased again when placed in the higher concentration (495.0µM).

The change in diameter was calculated by taking, firstly the diameter at the narrowest part of the fibre for each of the three treatments and secondly the diameter of the fibre during growth after addition of cysteine - deemed to be the in vitro diameter, and taking the ratio of these to the in vivo diameter. The results are shown in Figure 6 and demonstrate that in follicles where the cysteine concentration was reversed, the fibre diameter was similarly reversed.

## Claims

1. A cosmetic method for imparting body and volume to human hair comprising increasing the diameter of the hair fibre by supplementation of the hair root with a composition comprising between 0.01 and 10% by weight of the composition of cysteine, the cysteine being delivered to the hair root by topical application of the composition to the hair and/or the scalp characterised in that the source of the cysteine is a protein hydrolysate comprising amino acids in free and peptide form, wherein more than 50% by weight of the hydrolysate are free amino acids and at least 2% by weight of the hydrolysate of the amino acids are cysteine.

2. A method according to claim 1 in which the cysteine is used at a level of between 0.1 and 0.5% by weight of the composition.

3. A method according to any preceding claim in which the cysteine is delivered to the hair or scalp from a liquid base.

4. A method according to any preceding claim in which the composition is applied to the hair in a vehicle which contains a preservative and which is suitable to remain in contact with the hair and scalp for several hours.

5. A method according to any preceding claim in which the composition is in the form of a lotion that may be applied and left on overnight.

6. A method according to any preceding claim in which the vehicle is a liquid, gel or paste and includes shampoos, conditioners, hair sprays, styling gels and lotions.

## Patentansprüche

1. Kosmetisches Verfahren zum Verleihen von Fülle und Volumen dem menschlichen Haar, umfassend Erhöhen des Durchmessers der Haarfaser durch Ergänzen der Haarwurzel mit einer Zusammensetzung, umfassend zwischen 0,01 und 10 Gewichtsprozent der Zusammensetzung Cystein, wobei das Cystein durch örtliche Anwendung der Zusammensetzung auf das Haar und/oder die Kopfhaut an die Haarwurzel abgegeben wird, dadurch gekennzeichnet, dass die Cysteinquelle ein Proteinhydrolysat ist, umfassend Aminosäuren in freier und Peptidform, wobei mehr als 50 Gewichtsprozent des Hydrolysats freie Aminosäuren sind und mindestens 2 Gewichtsprozent des Hydrolysats der Aminosäuren Cystein sind.

2. Verfahren nach Anspruch 1, wobei das Cystein in einem Anteil zwischen 0,1 und 0,5 Gewichtsprozent der Zusammensetzung verwendet wird.

3. Verfahren nach einem vorangehenden Anspruch, wobei das Cystein aus einer flüssigen Grundlage an das Haar oder die Kopfhaut abgegeben wird.

4. Verfahren nach einem vorangehenden Anspruch, wobei die Zusammensetzung in einem Träger, der ein Konservierungsmittel enthält und der zum Beibehalten des Kontakts mit dem Haar und der Kopfhaut für einige Stunden geeignet ist, auf das Haar aufgetragen wird.

5. Verfahren nach einem vorangehenden Anspruch, wobei die Zusammensetzung in Form einer Lotion vorliegt, die aufgetragen und über Nacht belassen werden kann.

6. Verfahren nach einem vorangehenden Anspruch, wobei der Träger eine Flüssigkeit, ein Gel oder eine Paste ist und Shampoos, Konditionierungsmittel, Haarsprays, Frisiergele und Lotionen einschließt.

## Revendications

1. Procédé de traitement cosmétique pour transmettre corps et volume aux cheveux humains comprenant une augmentation du diamètre de la fibre du cheveu par supplémentation de la racine du cheveu avec une composition comprenant entre 0,01 et 10% en poids de cystéine par rapport à la composition, la cystéine étant délivrée à la racine du cheveu par application topique de la composition sur le cheveu et/ou le cuir chevelu, caractérisé en ce que la source de la cystéine est un hydrolysat de protéine comprenant des acides aminés sous forme libre et peptidique, dans lequel plus de 50% en poids de l'hydrolysat sont des acides aminés libres et au moins 2% en poids de l'hydrolysat des acides aminés sont la cystéine.

2. Procédé selon la revendication 1, dans lequel la cystéine est utilisée à une teneur comprise entre 0,1 et 0,5% en poids de la composition.

3. Procédé selon l'une quelconque des revendications précédentes, dans lequel la cystéine est délivrée sur le cheveu ou le cuir chevelu à partir d'une base liquide.

4. Procédé selon l'une quelconque des revendications précédentes, dans lequel la composition est appliquée sur le cheveu dans un véhicule qui contient un conservateur et qui est approprié pour rester en contact avec le cheveu ou le cuir chevelu pendant plusieurs heures.

5. Procédé selon l'une quelconque des revendications précédentes, dans lequel la composition est sous forme d'une lotion qui peut être appliquée et laissée une nuit.

6. Procédé selon l'une quelconque des revendications précédentes, dans lequel le véhicule est un liquide, un gel ou une pâte et inclut les shampoings, les conditionneurs, les laques, les gels coiffants et les lotions.
